Europäisches Patentamt

European Patent Office

Office européen des brevets

(11) Publication number: **0 062 892**
**B1**

# EUROPEAN PATENT SPECIFICATION

(45) Date of publication of patent specification: **16.10.85**

(21) Application number: **82102965.9**

(22) Date of filing: **07.04.82**

(51) Int. Cl.[4]: **G 01 N 33/573,**
**G 01 N 33/543, C 12 Q 1/50**

(54) **Single incubation immunochemical assay for creatin phosphokinase MB.**

(30) Priority: **13.04.81 US 253607**

(43) Date of publication of application:
**20.10.82 Bulletin 82/42**

(45) Publication of the grant of the patent:
**16.10.85 Bulletin 85/42**

(84) Designated Contracting States:
**AT BE CH DE FR GB IT LI LU NL SE**

(56) References cited:
**EP-A-0 042 755**
**EP-A-0 048 357**
**EP-A-0 049 898**
**FR-A-2 385 099**
**GB-A-1 348 938**
**GB-A-1 549 069**
**GB-A-1 563 299**
**US-A-4 067 775**
**US-A-4 123 343**

**J. of imm. Methods 42 (1981), pages 11-15**

(73) Proprietor: **AMERICAN HOECHST**
**CORPORATION**
**Route 202-206 North**
**Somerville, N.J. 08876 (US)**

(72) Inventor: **Amshey, Joseph William, Jr.**
**1012 Saint Albans Place**
**Leucadia California 92024 (US)**

(74) Representative: **Meyer-Dulheuer, Karl-Hermann,**
**Dr. et al**
**HOECHST Aktiengesellschaft Zentrale**
**Patentabteilung Postfach 80 03 20**
**D-6230 Frankfurt/Main 80 (DE)**

Courier Press, Leamington Spa, England.

## Description

### Background of the Invention

Solid-phase "Sandwich" immunoassay is a technique utilized to assay a ligand having at least two antibody combining sites or epitopes. This type of assay is currently one of the most sensitive methods used to determine biologically active components in plasma and other body fluids. The assay requires the interaction of three components: (1) the ligand to be assayed; (2) specific antibody directed against the ligand attached to an insoluble support material and (3) enzyme labeled antibody that is specific for the ligand. The methodology of the "Sandwich" assay involves (1) incubating the ligand with insolubilized specific antibody to produce an initial insolubilized antibody-ligand conjugate; (2) separating the initial conjugate and unreacted ligand; (3) incubating the initial conjugate with enzyme labeled antibody to produce a final insolubilized antibody-ligand-enzyme labeled antibody conjugate; (4) separating the final conjugate and unreacted enzyme labeled antibody; and (5) determining the enzyme activity of either the final conjugate (solid phase) or the separated unreacted enzyme labeled conjugate (liquid phase). The enzyme activity determined is related to the amount of ligand in the test fluid.

While the conventional "Sandwich" assay excels in sensitivity, it is time consuming and laborious to run due to the number of steps involved. When one attempts to reduce the number of steps in a conventional assay by incubating insolubilized antibody, ligand and enzyme labeled antibody in the presence of one another, enzyme labeled antibody tends to bind most of the available epitopes on the ligand and "Sandwich" formation is impaired. The tendency of the labeled antibody to monopolize or saturate the ligand occurs because the labeled antibody (i.e. solution antibody) has greater motility than the insolubilized antibody.

The present invention circumvents the problems inherent in the prior art with respect to assaying a liquid for the presence of creating phosphokinase MB by providing a method of determining that enzyme in a "Sandwich" assay which utilizes a single incubation.

### Summary of the Invention

The present invention relates to a diagnostic method for determining creatine phosphokinase MB (CK-MB) in a liquid medium.

In the invention, the determination of CPK-MB is accomplished utilizing a modified solid-phase "Sandwich" assay. The two antibody reagents employed in the assay method contain antibodies directed against different epitopes on the CPK-MB. The use of two antibodies having different binding specificities permits simultaneous incubation of insolubilized antibody, CPK-MB ("antigen"), and enzyme labeled antibody with resulting optimum sandwich geometry. Alignment of the antigen in the sandwich matrix is achieved in the present invention by employing polyclonal and/or monoclonal antibodies.

Accordingly, a first aspect of the invention relates to a method for determining CPK-MB in a liquid medium suspected of containing same, which comprises providing a reaction mixture comprising:

(i) liquid medium suspected of containing CPK-MB;

(ii) insolubilized first antibody reagent containing antibody having specificity for at least one epitope on one of the isoenzymes M or B; and

(iii) enzyme labeled second antibody reagent containing antibody having specificity for at least one epitope on the second of the isoenzymes M or B and that second antibody reagent being nonreactive with at least one epitope bindable by said first antibody reagent;

incubating said reaction mixture to form an insolubilized antibody-antigen-labeled antibody conjugate when CPK-MB is present in the liquid medium; separating the reaction mixture into a solid phase and a liquid phase; and determining the enzyme activity (of the label) of either the solid phase or the liquid phase whereby said activity is related to the amount of CPK-MB in said liquid medium.

A second aspect of the invention relates to the use of monoclonal specific antibodies in the first and second antibody reagents employed in the method taught herein.

Still another aspect of the invention relates to the use of polyclonal specific antibodies in the first and second antibody reagents employed in the method taught herein.

A further aspect of the present invention relates to the method taught herein wherein one of the first and second antibody reagents contains polyclonal specific antibody and the other reagent contains monoclonal specific antibody.

### Detailed Description

Both polyclonal and monoclonal specific antibodies may be employed in the process of the present invention.

Polyclonal specific antibodies are produced in an animal in response to immunization with a specific immunizing molecule (immunogen). Antibodies produced in this manner do not cross-react with other immunogens. A polyclonal antiserum contains a population of antibodies which is directed against a number of antigenic determinants (epitopes) on the immunogen.

Monoclonal specific antibodies are derived by fusing splenic lymphocytes obtained from an immunized host with malignant myeloma cells. Such fusion results in hybrid cells which express both the lymphocyte's property of specific antibody production and the immortal character of the myeloma cells. The progeny, or clone of a single hybrid cell secretes monoclonal antibody which is specifically directed against a single epitope on the immunogen.

Both of the above-described antibody types may be employed in the antibody reagents utilized in the present invention, i.e. polyclonal or monoclonal specific antibody may be independently employed in the first and second antibody reagents used herein provided that antibody in the first reagent and antibody in the second reagent recognize and bind different epitopes on CPK-MB.

When an antigen contains two different, isolatable, antibody generating subunits having non-shared epitopes, the antigen can be determined by reaction with a first antibody reagent containing antibody directed against epitopes on one subunit and a second antibody reagent containing antibody directed against epitopes on the other subunit.

In the case of an enzyme existing in the form of multiple isoenzymes composed of combinations of two subunits, the heterodimeric isoenzyme may be determined according to the present invention by employing a first antibody reagent containing antibody directed against one of the homodimeric isoenzymes and a second antibody reagent containing antibody directed against the other homodimeric isoenzyme. The enzyme creatine phosphokinase exists in the form of at least three isoenzymes designated CPK-MM, CPK-MB and CPK-BB. In the present invention, the heterodimeric isoenzyme (CPK-MB) is determined by employing antibody to one of the homodimeric isoenzymes (i.e. CPK-MM or CPK-BB) in the first antibody reagent and antibody to the other homodimeric isoenzyme in a second antibody reagent.

Both polyclonal and monoclonal specific antibodies against CPK-MB measurable by the present invention are available commercially or can be prepared by known procedures. See for example Hurn et al, "Production of Reagent Antibodies" in Method in Enzymology, 70, 104—142 (1980).

The first antibody reagent employed herein comprises appropriate antibody attached to an insoluble support. Suitable support materials that may be utilized include, for example, polypropylene, polystyrene, nylon, cellulose and the like. Polystyrene microtiter plates, nylon beads and nylon and cellulose fibers afford suitable support means for the attachment of antibody. The attachment of antibody to the support is not critical and may be accomplished by (1) covalent coupling of the antibody to the support material; (2) physical entrapment of the antibody in the support material; and (3) physical adsorption of the antibody on the support material.

The second antibody reagent employed herein comprises appropriate antibody labeled with a suitable enzyme. Enzymes suitable for use in the present invention include horseradish peroxidase, glucose oxidase, alkaline phosphatase, β-galactosidase, glucoamylase and the like. The enzyme labeled second antibody reagent of the present invention is prepared by covalent coupling of the selected enzyme to the antibody. Methods employed for coupling enzymes to antibodies include condensation of existing functional groups in the presence or absence of a coupling reagent. A useful method for coupling horseradish peroxidase (HRP) to proteins involves oxidizing the carbohydrate moiety of the enzyme with sodium periodate and thereafter allowing the resulting aldehyde groups to react with amino groups of the antibody. (Nakane et al, J. Histochim. Cytochem., 22, 1084 (1974). Alkaline phosphatase can be coupled to proteins by the condensation of free amino groups in the foregoing entities with aldehyde groups provided by a coupling reagent such as glutaraldehyde. Additional details on coupling methods are described in the art. See for example Kennedy et al, Clin. Chim. Acta, 70, 1 (1976).

The process of the present invention is carried out by simultaneously combining liquid medium suspected of containing CPK-MB, insolubilized first antibody reagent and enzyme labeled second antibody reagent. The resulting reaction mixture is incubated for approximately 1 to 4 hours at room temperature. After incubation, the liquid phase of the reaction mixture is separated from the insoluble phase and the enzyme activity of either phase is determined by a suitable detection reaction. The enzymatic activity of the insoluble phase will be directly related to the amount of ligand in the liquid test sample whereas the enzymatic activity of the liquid phase will be inversely related to the amount of ligand in the test sample.

The determination of enzymatic activity in the present invention is accomplished with a detection reaction system suitable for the particular enzyme employed. Since there are differences, not only between assays for different enzymes, but even in the variety of assays for a particular enzyme, no general description of the assay techniques can be given; however, a wide diversity of media, conditions and substrates suitable for enzymes employed herein can be found in Bergmeyer, Methods of Enzymatic Analysis, Academic Press, New York, 1965.

In order to obtain quantitative results using the process of the present invention, the quantity of the insolubilized first antibody reagent mixed with test liquid and enzyme labeled second antibody reagent should generally be larger than the quantity which is capable of forming bonds with the total amount of CPK-MB assumed to be in the test liquid. The quantity, in practice, is chosen in conformity with the aforementioned criteria based on the assumption that the antigen is present in the liquid medium at the highest concentration possible.

The liquid medium suspected of containing CPK-MB may be a natural or synthetic liquid. In many instances, the liquid will be a biological fluid. Biological fluids which may be examined for the antigen according to the process of the present invention include, for example, serum, plasma, urine, amniotic fluid and cerebrospinal fluid.

## Description of Specific Embodiments

The following specific description is given to enable those skilled in the art to more clearly understand and practice the present invention. It should not be considered as a limitation upon the scope of the invention but merely as being illustrative and representative thereof.

### Example

The following example illustrates the determination of creatine phosphokinase MB using the process of the present invention.

#### 1. Reagents

(a) *Insolubilized first antibody reagent*

Rabbit antibody specific for human CPK-BB isoenzyme adsorbed onto the walls of wells in a polystyrene microtiter plate.

(b) *Enzyme labeled second antibody reagent*

Rabbit antibody specific for human CPK-MM covalently bound to horseradish peroxidase in a predetermined dilution in 0.05 M TRIS® buffer, pH 7.4, containing 5 g/l hemaccael, 3 mM phenol, 0.3 g/l rabbit gammaglobulin, 30% bovis serum, 20 g/l TWEEN® 20 and 2.5 g/l genamin®-0—100.

(c) *Buffer/Substrate*

0.2 M Citrate phosphate buffer, pH 4.8, containing 2 mg/ml o-phenylenediamine, 12.5 g/l $KH_2PO_4$, 0.82 g/l urea peroxide and 0.1 g/l thimerosal.

(d) *Stopping solution*

2.5 M sulfuric acid

(e) *Washing solution*

0.01 M Phosphate buffer, pH 6.5, containing 10 g/l NaCl and 0.2 g/l TWEEN® 20.

(f) *CPK-MB Standards*

A dilution series of human CPK-MB (1.0, 5.0, 10.0, 25.0 and 50.0 ng/ml) in 0.01 M phosphate puffer, pH 6.5, containing 10 g/l NaCl.

#### 2. Assay Protocol

Two separate wells of the polystyrene microtiter plate coated with rabbit antibody specific for human CPK-BB are used for each test sample and each standard. 100 µl of test sample or standard is dispensed into a well followed by 100 µl of enzyme labeled second antibody reagent. The plate is then incubated at 25°C for 2 hours. Thereafter the wells are emptied by aspiration and washed thoroughly with washing solution. After washing, 200 µl of buffer substrate is added to each well and the plate is incubated at 25°C. After 30 minutes, 50 µl of stopping solution is added to each well. The color developed in each well is determined by measuring the absorbance at 492 nm. The amount of CPK-MB in the test sample is determined by comparing the absorbance of the test sample with the absorbance of the standards.

## Claims

1. A *method of determining creatine phosphokinase MB in a liquid medium suspected of containing same, which comprises:

    (a) providing a reaction mixture comprising:

    (i) liquid medium suspected of containing creatine phosphokinase MB;

    (ii) insolubilized first antibody reagent containing antibody having specificity for at least one epitope on one of the isoenzymes M or B and

    (iii) enzyme labeled second antibody reagent containing antibody having specificity for at least one epitope on the second of the isoenzymes M or B and that second antibody reagent being nonreactive with at least one epitope bindable by said first antibody reagent;

    (b) incubating said reaction mixture to form an insolubilized antibody-antigen-labeled antibody conjugate when creatine phosphokinase MB is present in said liquid medium;

    (c) separating said reaction mixture into a solid phase and a liquid phase; and

    (d) determining the enzyme activity of either the solid phase or the liquid phase whereby said activity is related to the amount of creatine phosphokinase MB in said liquid medium.

2. A method according to Claim 1 wherein antibody contained in said first antibody reagent is rabbit anti-human creatine phosphokinase BB.

3. A method according to Claim 1 wherein antibody contained in said second antibody reagent is rabbit anti-human creatine phosphokinase MM.

## Revendications

1. Une méthode de dosage de la créatine phosphokinase MB dans un milieu liquide présumé contenir cette enzyme, méthode dans laquelle:

    (a) on forme un mélange de réaction comprenant:

    (1) le milieu liquide présumé contenir la CPK-MB,

    (2) un premier réactif d'anticorps insolubilisé contenant un anticorps spécifique pour au moins un épitope de l'une des iso-enzymes M ou B, et

    (3) un second réactif d'anticorps marqué par une enzyme, qui contient un anticorps ayant une spécificité pour au moins un épitope de la seconde des iso-enzymes M et B, ce second réactif ne réagissant pas avec au moins un épitope liable par le premier réactif;

    (b) on fait incuber ce mélange réactionnel pour former un conjugué anticorps insolubilisé-antigène-anticorps marqué si la CPK-MB se trouve dans le milieu liquide;

    (c) on sépare le mélange réactionnel en une phase solide et une phase liquide; et

    (d) on mesure l'activité enzymatique de la phase solide ou de la phase liquide, activité qui est en rapport avec la quantité de CPK-MB dans le milieu liquide.

2. Méthode selon la revendication 1 dans laquelle l'anticorps du premier réactif d'anticorps est la créatine phosphokinase BB de lapin antihumaine.

3. Méthode selon la revendication 1 dans laquelle l'anticorps du second réactif d'anticorps est la créatine phosphokinase MM de lapin antihumaine.

**Patentansprüche**

1. Ein Verfahren zur Bestimmung von Creatin-Phosphokinase MB in einer diese enthaltenden Flüssigkeit, dadurch gekennzeichnet, daß

a) eine Reaktionsmischung hergestellt wird, die

(i) aus der die Creatin-Phosphokinase MB enthaltenden Flüssigkeit;

(ii) einem ersten, unlöslichen Antikörperreagenz, das für mindestens ein Epitop auf einem der Isoenzyme M oder B spezifische Antikörper enthält und

(iii) einem zweiten enzymmarkierten Antikörperreagenz, das für mindestens ein Epitop an dem zweiten der Isoenzyme M oder B spezifische Antikörper enthält und wobei dieses zweite Antikörperreagenz mit mindestens einem der durch das erste Antikörperreagenz bindungsfähigen Epitope nicht reagiert, besteht;

b) diese Reaktionsmischung inkubiert wird, wobei ein Komplex aus dem unlöslichen Antikörper, dem Antigen und dem markierten Antikörper gebildet wird, falls Creatin-Phosphokinase MB in der Flüssigkeit vorhanden ist;

c) die Reaktionsmischung in eine feste und eine flüssige Phase getrennt wird; und

d) die Enzymaktivität entweder in der festen oder der flüssigen Phase bestimmt wird, wobei diese Aktivität in Beziehung steht zu der Menge an Creatin-Phosphokinase MB in jener Flüssigkeit.

2. Ein Verfahren nach Anspruch 1, worin die in jenem ersten Antikörperreagenz enthaltenen Antikörper solche gegen humane Creatin-Phosphokinase BB gerichteten vom Kaninchen sind.

3. Ein Verfahren nach Anspruch 1, worin die in jenem zweiten Antikörperreagenz enthaltenen Antikörper solche gegen humane Creatin-Phosphokinase MM gerichteten vom Kaninchen sind.